# EUROPÄISCHE PATENTSCHRIFT

(11) **EP 2 599 512 B1**
(45) Veröffentlichungstag und Bekanntmachung des Hinweises auf die Patenterteilung: **11.05.2016**
(21) Anmeldenummer: 11401648.8
(22) Anmeldetag: 01.12.2011
(51) Int. Cl.: A61M 15/00, A61M 11/04

(54) **Heißluftextraktionsinhalator mit kombinierter Luft- und Strahlungsheizung**
Warm air extraction inhaler with combined air and radiant heating
Inhalateur d'extraction à air chaud doté d'un chauffage combinant air et rayonnement

(43) Veröffentlichungstag der Anmeldung: 05.06.2013
(73) Patentinhaber: Stobi GmbH & Co. KG, 78532 Tuttlingen (DE)
(72) Erfinder: Storz, Markus, 78532 Tuttlingen (DE)
(74) Vertreter: Späth, Dieter

(56) Entgegenhaltungen:
- EP-A1- 0 858 744
- EP-A1- 2 210 637
- WO-A1-99/11311
- US-A1- 2003 217 750

## Beschreibung

Die Erfindung betrifft einen Heißluftextraktionsinhalator, mit einer Heizung zur Erwärmung von unter Wärmeeinwirkung aerosolbildende Substanzen die von Luft durchströmt in die Luft übergehen und entweder über einen Zwischenspeicher oder direkt aus dem Inhalator über eine Inhalationsleitung mit Mundstück heraus inhaliert werden.

Derartige therapeutische Verdampfungsvorrichtungen zur Erzeugung von Aroma- und/oder Wirkstoffdämpfen für die Inhalation sind aus dem Stand der Technik in vielfältigen Ausführungsformen bekannt. Beispielhaft wird auf die Patentschriften DE 100 42 396 B4 und DE 198 03 376 C1 verwiesen. Die dort beschriebenen Inhalatoren dienen zur thermischen Verdampfung und anschließenden Inhalation von verdampfbaren, also unter Wärmeeinwirkung aerosolbildenden Substanzen.

Einfache Heißluftextraktionsinhalatoren verfügen lediglich über eine beheizte Füllkammer zur Erwärmung der zu verdampfenden Substanzen mittels Wärmestrahlung. Diese Bauweise ist kostengünstig, ermöglicht aber nur eine unzureichende Verdampfung, da während der Inhalation die nachströmende unbeheizte Luft die Füllkammer und die darin enthaltenen Substanzen wieder abkühlt und dadurch den Verdampfungsvorgang unterbricht. Es muss dann gewartet werden bis die Strahlungswärme der Füllkammer die enthaltenen Substanzen wieder für die Verdampfung hinreichend erwärmt hat. Heißluftextraktionsinhalatoren, die lediglich mit Wärmestrahlung arbeiten, eignen sich allenfalls für die Verdampfung von Reinstoffen, für die Verdampfung von Aromen und Wirkstoffen aus Heilkräutern sind sie nur eingeschränkt nutzbar.

Wesentlich ergiebiger ist die Methode, bei der die Substanzen mit einem heißem Luftstrom beaufschlagt werden, der beim Passieren eines Wärmeerzeugers aufgeheizt wird. Die Heißluft durchströmt die Füllkammer mit den Substanzen, wobei die Substanzen verdampfen oder ihre Wirkstoffe wie z.B. bei Heilkräutern aus einem Substrat heraus ausdünsten (extrahieren), die dann in die Heißluft übergehen. Diese zum Beispiel mit Aromen und/oder Wirkstoffen befrachtete Heißluft wird als Einatemluft inhaliert, nachdem sie auf eine zum Einatmen angenehme Temperatur heruntergekühlt wurde, wobei die Wirkstoffe nach dem Inhalieren über die Lunge in den Blutkreislauf gelangen. Für die Heißluftextraktion werden häufig Heilkräuter oder andere geeignete pflanzliche Substanzen verwendet, die dem Verwendungszweck entsprechend angemessen zerkleinert sind oder aber auch synthetische Substanzen mit therapeutischen Wirkstoffen, die in pulverisierter Form vorliegen. Es können auch flüssige Substanzen verdampft werden. Zur Aerosolbildung müssen diese Substanzen mit Heißluft von beispielsweise 200° C beaufschlagt werden. Einen Verdampfer, der in einem Wärmetauscher Heißluft erzeugt, die durch eine Füllkammer hindurchgeführt wird und in der Füllkammer enthaltene Substanzen erwärmt, offenbart die Europäische Patentanmeldung EP 2 210 637 A1.

Die europäische Patentanmeldung EP 0 858 744 A1 offenbart einen Heißluftextraktionsinhalator mit einem Rohr, das durch einen gasbeheizbaren Heizkanal steckbar ist. In dem Bereich, der sich in dem Heizkanal befindet, enthält das Rohr einen zylindrischen Körper, der mit Durchgangslöchern durchsetzt ist und der zu verdampfende Substanzen enthält. Luft, die durch das Rohr gesaugt wird, erwärmt sich in den Durchgangslöchern und verdampft die enthaltenen Substanzen. Ein großer Nachteil des bekannten Verdampfers ist, dass die Luft kalt in die Durchgangslöcher des die verdampfbaren Substanzen enthaltenden Zylinders strömt. Eine Verdampfung erfolgt erst, wenn die Luft ausreichend erwärmt worden ist, also erst in einem in Strömungsrichtung hinteren Bereich des zylindrischen, die verdampfbaren Substanzen enthaltenen Zylinders. In einem in Strömungsrichtung vorderen Bereich wird nicht verdampft, weil die Luft kalt einströmt.

Bei der effektiveren Methode der Lufterwärmung wird üblicherweise ein Wärmeerzeuger verwendet, der mindestens einen Luftkanal für den zu erwärmenden Luftstrom aufweist. Der Luftkanal kann den Wärmeerzeuger innen durchsetzen oder außen einschließen. Es gibt auch Ausführungen, bei denen elektrische Heizdrähte offen im Luftkanal angebracht sind. Prinzipiell gibt es bei bekannten gattungsgemäßen Heißluftextraktionsinhalatoren zwei Grundprinzipien zur Erzeugung des heißen Luftstroms mittels des Wärmeerzeugers. Zum einen kann der Luftstrom durch Ansaugen von Luft an einer Austrittsöffnung oder zum anderen durch Einblasen von Luft in eine Eintrittsöffnung des Luftkanals erzeugt werden, wobei der erforderliche Über- bzw. Unterdruck mittels Lungenkraft oder unter Verwendung eines Gebläses oder einer Pumpe, beispielsweise einer Membranpumpe, aufgebaut werden kann. Durch den thermischen Kontakt mit dem Wärmeerzeuger wird die an einem Inhalatoreinlass in den Luftkanal einströmende Umgebungsluft in Abhängigkeit von der von dem Wärmeerzeuger ausgehenden Temperatur und dem Volumenstrom durch den Luftkanal von Zimmertemperatur auf beispielsweise 200° C erwärmt. Die Temperatur ist abgestimmt auf den Siede- oder Extraktionspunkt der für die Inhalation verwendeten aerosolbildenden Substanzen, so dass deren Wirkstoffe und/oder Aromen beim Durchtritt des Heißluftstroms durch die Füllkammer verdampfen und die Aerosole von dem Luftstrom aufgenommen werden. Nach dem Passieren der Füllkammer mit den aerosolbildenden Substanzen steht der aerosolbefrachtete Luftstrom als Einatemluft zur Inhalation zur Verfügung.

Zunächst aber muss der heiße Luftstrom sowohl die kalten Substanzen als auch die kalte umgebende Füllkammer aufheizen, damit eine Verdampfung dieser Substanzen überhaupt stattfinden kann.

Bei Verwendung eines Heißluftextraktionsinhalators mit Pumpe und Zwischenspeicher, beispielsweise eines Kunststoffinhalationsbeutels wie in DE 198 03 376 C1 beschrieben, ergibt sich kein spürbarer Nachteil, da sich dort die zunächst nicht angereicherte Inhalationsluft später mit der mit Aromen und/oder Wirkstoffen angereicherten Inhalationsluft vermischt, bevor sie aus dem Zwischenspeicher heraus inhaliert wird. Die Anwendung der hier beschriebenen Erfindung würde aber auch bei einem Heißluftextraktionsinhalator mit Pumpe und Zwischenspeicher zu einer effektiveren Verdampfung führen.

Im Unterschied dazu ergibt sich bei Verwendung eines Heißluftextraktionsinhalators mit Heißluftstrom, bei dem direkt aus dem Inhalator heraus inhaliert bzw. angesaugt wird, das Problem, dass zunächst einige Atemzüge benötigt werden, um sowohl die kalten Substanzen als auch die kalte umgebende Füllkammer mittels Heißluftstrom aufzuheizen, da vorher keine Verdampfung stattfinden kann. Dies ist für den Anwender nicht nur unkomfortabel, er weiß auch nicht genau wann die Verdampfung einsetzt.

Ausgehend von dem Stand der Technik liegt der Erfindung die Aufgabe zugrunde, eine Möglichkeit vorzuschlagen, wie der Anwender von Heißluftextraktionsinhalatoren, bei denen direkt aus dem Inhalator heraus inhaliert wird, möglichst sofort mit dem ersten Inhalationszug und dann andauernd Aroma- und wirkstoffreiche Dämpfe erhalten kann.

Diese Aufgabe wird erfindungsgemäß durch einen Heißluftexktrationsinhalator mit den Merkmalen des Anspruchs 1 gelöst. Weitere vorteilhafte Ausgestaltungen sind den Unteransprüchen zu entnehmen.

Kerngedanke der Erfindung ist es, die Heizung von Heißluftextraktionsinhalatoren so zu verbessern, dass sowohl eine sofortige, als auch während des Inhalationszuges andauernde Verdampfung stattfinden kann.

Danach weist der erfindungsgemäße Heißluftexktrationsinhalator eine Heizung auf, die sowohl eine Füllkammer mit Inhalt beheizt, als auch in der Lage ist, einen heißen Luftstrom zu erzeugen, welcher durch die Füllkammer mit Inhalt hindurchgeführt wird. Dazu weist die Heizung, die als massiver Wärmetauscher mit einem oder mehreren durchgeführten Luftkanälen ausgeführt sein kann, vorzugsweise in einer Ausführungsform eine im Wärmetauscher integrierte Füllkammer und in einer anderen bevorzugten Ausführungsform eine abnehmbare, aber in aufgesetztem Zustand über eine Kontaktfläche thermisch mit dem Wärmetauscher verbundene Füllkammer auf. Die Innenwand der Füllkammer ist in beiden Fällen so ausgebildet, dass der Füllkammerinhalt mittels Strahlungswärme aufgeheizt wird. Die beheizte Innenwand der Füllkammer erwärmt ihren Inhalt durch Strahlungswärme und am Rand durch thermischen Kontakt des Inhalts mit der Innenwand der Füllkammer unabhängig davon, ob Heißluft durch die Füllkammer strömt oder nicht. Die Beheizung der Füllkammer reicht aus, um in ihr enthaltene Substanzen auf eine zur aerosolbildende Temperatur zu erwärmen und auf dieser Temperatur zu halten, wenn die Füllkammer nicht von Luft durchströmt wird. Wird die Füllkammer von Luft durchströmt, ist diese zuvor aufgeheizt worden, so dass sie den Inhalt der Füllkammer nicht kühlt, sondern auf der zur aerosolbildenden Temperatur hält bzw. gemeinsam mit der Füllkammer auf diese Temperatur erwärmt.

Auch wenn die Erfindung auf Heißluftextraktionsinhalatoren zur direkten Inhalation eines Anwenders gerichtet ist, ist sie nicht auf solche Heißluftexktrationsinhalatoren beschränkt sondern allgemein auf gattungsgemäße Heißluftextraktionsinhalatoren gerichtet. Die Erfindung ist beispielsweise auch für Heißluftextraktionsinhalatoren von Vorteil, die einen Zwischenspeicher, in den das Aerosol/Luftgemisch zunächst gepumpt und aus dem dann später heraus inhaliert wird, aufweisen.

Die Füllkammer besteht aus einem inneren, gut wärmeleitendem Teil aus z.B. Metall, das insbesondere die Innenwand der Füllkammer bzw. ein inneres Kammergehäuse bildet, und einem äußeren, gut wärmeisolierendem umlaufenden Teil aus z.B. Kunststoff. Bei der im Wärmetauscher integrierten Füllkammer ist das innere Kammergehäuse Bestandteil des Wärmetauschers.

Bei der abnehmbaren Füllkammer ist das wärmeleitende innere Kammergehäuse zum Beispiel stirnseitig an dem Wärmetauscher anlegbar. Dies ermöglicht während des Aufheizvorgangs oder auch bei bereits aufgeheiztem Wärmetauscher einen schnellen Wärmeübergang von dem Wärmetauscher zu dem inneren Kammergehäuse mittels direkter Wärmeleitung. Dabei besteht das innere Kammergehäuse, das die Füllkammer für die zu verdampfenden Substanzen umgibt und begrenzt, aus einem gut wärmeleitenden Material wie z. B. Metall.

Bei im Wärmetauscher integriertem oder anliegendem inneren Kammergehäuse trocknen in der Füllkammer aufgenommene feuchte Substanzen, beispielsweise Pflanzenmaterial, bevor mit der Inhalation begonnen wird noch während der Aufheizphase. Das Wasser in dem Pflanzenmaterial verdunstet dadurch in kurzer Zeit, typischerweise in weniger als einer Minute: Nach dem Trocknen des Pflanzenmaterials kann anschließend das Pflanzenmaterial oder allgemein die in der Füllkammer enthaltenen, durch Wärmeeinwirkung Aerosol bildenden Substanzen mittels dem durch Lungenkraft erzeugten Heißluftstrom aus dem Pflanzenmaterial freigesetzt und von der Einatemluft aufgenommen werden. Solange Feuchtigkeit in den Substanzen enthalten ist, verhindert sie eine Erwärmung über den Siedepunkt des Wassers. Feuchtigkeit verhindert die Erwärmung der Substanzen auf die zur Aerosolbildung notwendige Temperatur.

In einer bevorzugten Ausführungsform der Erfindung ist das innere Kammergehäuse zylindrisch ausgebildet und axial bewegbar in dem Inhalatorgehäuse geführt. Dies ist insbesondere dann von Vorteil, wenn der Wärmetauscher ebenfalls eine im Wesentlichen zylinderförmige Kontur aufweist. Damit kann das innere Kammergehäuse mit der Füllkammer auf einfache Weise stirnseitig und koaxial zu dem Luftkanal an dem Wärmetauscher derart angeordnet werden, dass der aus dem Wärmetauscher austretende Luftstrom durch Luftdurchtrittsöffnungen in einem Boden und einem Deckel der Füllkammer durch die darin befindlichen für Heißluftextraktion vorgesehenen Substanzen geleitet werden kann. Der Boden und/oder der Deckel der Füllkammer kann auch in üblicher Weise von einem metallischen Drahtgeflecht gebildet sein.

In einer vorteilhaften Ausführungsform des erfindungsgemäßen Heißluftextraktionsinhalators weist die abnehmbare Füllkammer umfangsseitig ein Gewinde auf, das mit einem Gegengewinde des Inhalatorgehäuses zusammen wirkt. Das Gewinde kann an einem Innenumfang oder an einem Außenumfang der Füllkammer ausgebildet sein, wobei das Gegengewinde des Inhalatorgehäuses jeweils komplementär dazu ausgebildet und korrespondierend dazu angeordnet ist. Das Drehen der Füllkammer gegenüber dem Inhalatorgehäuse des Heißluftextraktionsinhalators bewirkt abhängig von der Drehrichtung einen guten Kraftschluss und damit eine gute Dichtigkeit des inneren Kammergehäuses zu dem Wärmetauscher. Dasselbe gilt für den Füllkammerdeckel, welcher unabhängig von integrierter oder abnehmbarer Füllkammer in derselben Weise mit der Füllkammer verbunden wird.

Des Weiteren weist das äußere Kammergehäuse in einer bevorzugten Ausführungsform der Erfindung einen thermisch isolierenden Betätigungshebel zum Drehen des Kammergehäuses bzw. der Füllkammer gegenüber dem Inhalatorgehäuse auf. Damit werden Verbrennungen beim Abnehmen bzw. Aufsetzen der Füllkammer zu dem aufgeheizten Wärmetauscher sicher vermieden.

Bei einer zweckmäßigen Ausführungsform des erfindungsgemäßen Heißluftextraktionsinhalators ist das Inhalatorgehäuse nach Art eines Handgerätes mit einem Griff ausgebildet. Dabei kann ein den Wärmetauscher umschließender Inhalatorgehäusebereich als Griff ausgebildet sein oder das Inhalatorgehäuse weist einen seitlich angeordneten Griff auf.

Nachfolgend wird die Erfindung anhand eines in der Zeichnung dargestellten Ausführungsbeispiels näher erläutert. Weitere Merkmale der Erfindung ergeben sich aus der folgenden Beschreibung des Ausführungsbeispiels der Erfindung in Verbindung mit den Ansprüchen und der beigefügten Zeichnung. Die einzelnen Merkmale können für sich allein oder zu mehreren bei unterschiedlichen Ausführungsformen der Erfindung verwirklicht sein. Es zeigen:
- Figur 1: einen erfindungsgemäßen Heißluftextraktionsinhalator mit abnehmbarer Füllkammer in Seitenansicht;
- Figur 2: den Heißluftextraktionsinhalator gemäß Figur 1 in einer Achsschnittdarstellung;
- Figur 3: den Inhalatorauslass aus Figur 2 in einer Ausschnittsvergrößerung. und
- Figur 4: einen Inhalatorauslass mit integrierter Füllkammer in einer Ausschnittsvergrößerung

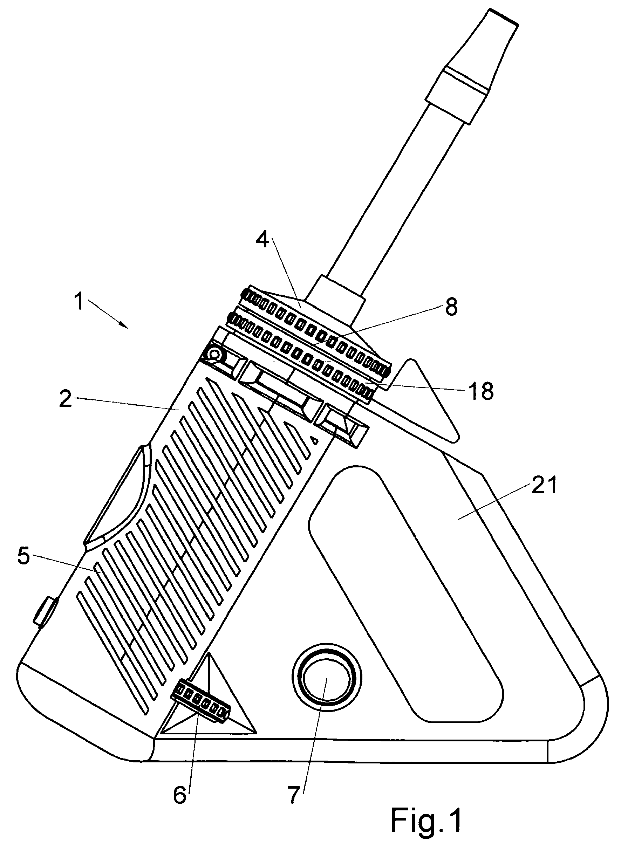

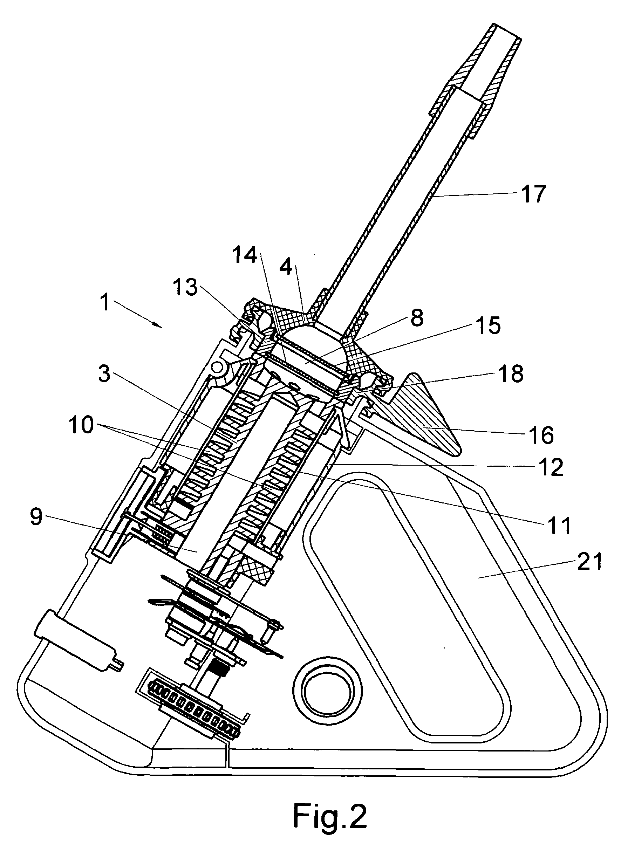

Die Figur 1 zeigt ein Ausführungsbeispiel eines erfindungsgemäßen Heißluftextraktionsinhalators 1 mit einem Inhalatorgehäuse 2, in dem ein zu dem Inhalatorgehäuse 2 hin wärmeisolierter Wärmetauscher 3 (Figur 2) zum Erwärmen eines Luftstroms angeordnet ist. Bei Beaufschlagung eines Inhalatorauslasses 4 des Heißluftextraktionsinhalators 1 mit Unterdruck, also beispielsweise bei einem Einatmen von Luft aus dem Heißluftextraktionsinhalator 1 durch den Inhalatorauslass 4, strömt Luft durch Belüftungsschlitze 5 in das Inhalatorgehäuse 2 und tritt anschließend von dort in den Wärmetauscher 3 ein. Nach dem Durchströmen des Wärmetauschers 3 und der abnehmbaren Füllkammer 8 tritt die Luft an dem Inhalatorauslass 4 wieder aus. Der Wärmetauscher 3 ist elektrisch beheizt, wobei dessen Temperatur mit einem Temperaturwähler 6 wählbar und dessen Versorgungsspannung über einen Netzspannungsschalter 7 schaltbar ist.

Figur 2 zeigt das Innere des Inhalatorgehäuses 2 in einer Achsschnittdarstellung. Als wesentliche Teile weist das Inhalatorgehäuse 2 den Wärmetauscher 3 und eine Füllkammer 8 für unter Wärmeeinwirkung Aerosol bildende Substanzen auf, die in Strömungsrichtung in Verlängerung des Wärmetauschers 3 zwischen dem Inhalatorauslass 4 und dem Wärmetauscher 3 angeordnet ist. Der Wärmetauscher 3 weist eine zentrale elektrische Heizpatrone 9 sowie zwei gewendelte Luftkanäle 10 auf, die umfangsseitig außen von einem Außenrohr 11 abgeschlossen sind. Das Außenrohr 11 ist von einem Isoliermantel 12 zur thermischen Isolierung des Wärmetauschers 3 gegenüber dem Inhalatorgehäuse 2 umgeben. Die Heizpatrone 9 und der Wärmetauscher 3 bilden zusammen mit dem Außenrohr 11 eine Heizung des Heißluftextraktionsinhalators 1.

Die Füllkammer 8 weist ein inneres Kammergehäuse 13 mit einem Kammerboden 14 und den Inhalatorauslass 4 mit einem Kammerdeckel 15 auf, wobei der Inhalatorauslass 4 zum Befüllen und zum Entleeren der Füllkammer 8 von dem äußeren Kammergehäuse 18 lösbar ist. Die Füllkammer 8 ist koaxial zu dem Wärmetauscher 3 angeordnet und in axialer Richtung in dem Inhalatorgehäuse 2 axial bewegbar geführt. Die Füllkammer 8 ist mittels einem thermisch isolierenden Betätigungshebel 16 abnehmbar. Der Inhalatorauslass 4 ist glockenförmig und wärmeisolierend ausgebildet. Er trägt den Kammerdeckel 15, der genau wie der Kammerboden 14 luftdurchlässig ausgeführt, beispielsweise perforiert ist, und einen aus dem Inhalatorgehäuse 2 vorstehenden Saugschlauch 17 zum Einatmen von Luft aus dem Heißluftextraktionsinhalator 1 (Inhalieren) aufweist.

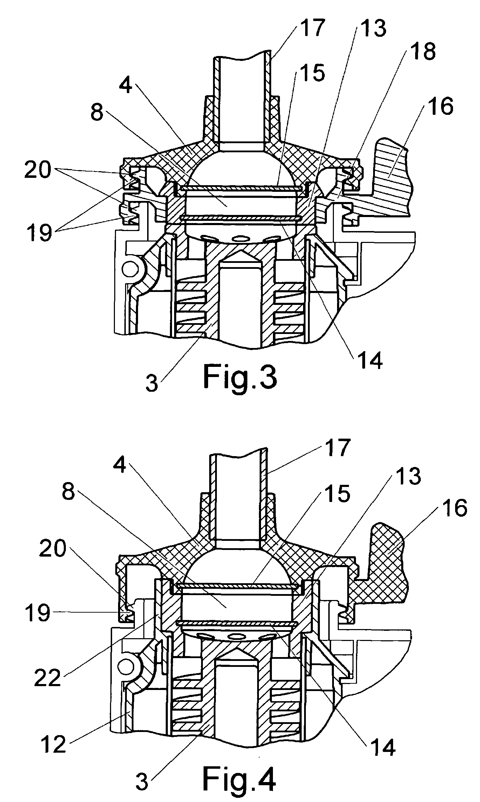

Die Figur 3 zeigt eine Ausschnittsvergrößerung des dem Inhalatorauslass 4 zugewandten Endes des Wärmetauschers 3 sowie die in Strömungsrichtung danach angeordnete Füllkammer 8 mit dem darauf folgenden Inhalatorauslass 4. Deutlicher als in der Figur 2 ist die Füllkammer 8 mit dem inneren Kammergehäuse 13, dem äußeren Kammergehäuse 18, dem Kammerboden 14 und dem Kammerdeckel 15 zu sehen.

Die Füllkammer 8 ist durch Abnehmen des Inhalatorauslasses 4 zusammen mit dem Saugschlauch 17 und dem Kammerdeckel 15 von dem inneren und äußeren Kammergehäuse 13 und 18 öffenbar. Die Figur 3 zeigt das innere Kammergehäuse 13 stirnseitig in Anlage an den Wärmetauscher 3, wobei ein direkter thermischer Kontakt zwischen dem aufgeheizten Wärmetauscher 3 und dem inneren Kammergehäuse 13 besteht, so dass sich das innere Kammergehäuse 13 über Wärmeleitung erwärmt und eine sich in der Füllkammer 8 befindliche, in der Zeichnung nicht dargestellte Aerosol bildende Substanz mittels Wärmestrahlung, ausgehend von dem inneren Kammergehäuse 13 erwärmt.

Die Füllkammer 8 kann in dieser bevorzugten Ausführungsform zur bequemeren Entleerung, Reinigung und Befüllung in axialer Richtung von dem Wärmetauscher 3 abgenommen werden. Dazu wird das Kammergehäuse 13 mittels des radial abstehenden Betätigungshebels 16, der an dem äußeren Kammergehäuse 18 angreift, gegenüber dem Wärmetauscher 3 und dem Inhalatorgehäuse 2 in Umfangsrichtung der Füllkammer 8 gedreht werden. Das innere Kammergehäuse 13 weist ein form- und/oder kraftschlüssig verbundenes äußere Kammergehäuse 18 auf, an dem der Betätigungshebel 16 angeformt und damit drehfest verbunden ist. Das äußere Kammergehäuse 18, welches das innere Kammergehäuse 13 umgreift, weist auf der zum Inhalatorgehäuse 2 gerichteten Außenumfangsseite ein Gewinde 19 auf, das bei dem dargestellten Ausführungsbeispiel von Wendelflügeln gebildet ist. Die Wendelflügel wirken mit einem zugeordneten Innengewinde 20 im Inhalatorgehäuse 2 zusammen. Abhängig von einer Bewegungsrichtung des Betätigungshebels 16 in oder entgegen dem Uhrzeigersinn bewegt sich die Füllkammer 8 zu dem Wärmetauscher 3 hin oder von ihm weg.

Die Figur 4 zeigt ebenfalls einen Ausschnitt des dem Inhalatorauslass 4 zugewandten Endes des Wärmetauschers 3 sowie die in Strömungsrichtung danach angeordnete Füllkammer 8 mit dem darauf folgenden Inhalatorauslass 4. Im Unterschied zur Figur 3 zeigt die Figur 4 ein im Wärmetauscher 3 integriertes inneres Kammergehäuse 13 der Füllkammer 8. Der Wärmetauscher 3 und das innere Kammergehäuse 13 bilden hier ein gemeinsames, einstückig ausgeführtes Teil. Das äußere Kammergehäuse 18 entfällt bei Figur 4 und wird durch einen Schutzzylinder 22 ersetzt, welcher zusammen mit dem Isoliermantel 12 gemeinsam einstückig ausgeführt ist. Der Betätigungshebel 16 ist hier mit dem Inhalatorauslass 4 gemeinsam einstückig ausgeführt.

Die Füllkammer 8 ist durch Abnehmen des Inhalatorauslasses 4 zusammen mit dem Saugschlauch 17 und dem Kammerdeckel 15 von dem inneren Kammergehäuse 13 öffenbar. Die Figur 4 zeigt das innere Kammergehäuse 13 zusammen als ein Stück mit dem Wärmetauscher 3, wobei eine direkte thermische Verbindung zwischen dem aufgeheizten Wärmetauscher 3 und dem inneren Kammergehäuse 13 besteht, so dass sich das innere Kammergehäuse 13 über Wärmeleitung erwärmt und eine sich in der Füllkammer 8 befindliche, in der Zeichnung nicht dargestellte Aerosol bildende Substanz mittels Wärmestrahlung, ausgehend von dem inneren Kammergehäuse 13 und zum Teil auch vom Wärmetauscher 3 erwärmt.

Der Inhalatorauslass 4 kann in dieser Ausführungsform in axialer Richtung von der Füllkammer 8 abgenommen werden. Dazu wird der Inhalatorauslass 4 mittels des radial abstehenden Betätigungshebels 16, der an dem Inhalatorauslass 4 angreift, gegenüber dem Wärmetauscher 3 und dem Inhalatorgehäuse 2 in Umfangsrichtung der Füllkammer 8 gedreht. Der Inhalatorauslass 4 ist mit dem Betätigungshebel 16 drehfest verbunden. Der Inhalatorauslass 4 weist auf der zum Inhalatorgehäuse 2 gerichteten Außenumfangsseite ein Gewinde 19 auf, das bei dem dargestellten Ausführungsbeispiel von Wendelflügeln gebildet ist. Die Wendelflügel 19 wirken mit einem zugeordneten Innengewinde 20 im Inhalatorgehäuse 2 zusammen. Abhängig von einer Bewegungsrichtung des Betätigungshebels 16 in oder entgegen dem Uhrzeigersinn bewegt sich die Füllkammer 8 zu dem Wärmetauscher 3 hin oder von ihm weg.

Der in den Figuren 1 bis 4 dargestellte erfindungsgemäße Heißluftextraktionsinhalator 1 ist vorzugsweise als Stand- und als Handgerät einsetzbar. Für die Verwendung als Handgerät ist das Inhalatorgehäuse 2 nach Art eines Handgerätes mit einem Griff 21 auf einer Seite des Inhalatorgehäuses 2 ausgebildet.

## Patentansprüche

1. Heißluftextraktionsinhalator (1), mit einem Wärmetauscher (3), der in der Lage ist, einen heißen Luftstrom zu erzeugen, mit einer Füllkammer (8) zur Befüllung mit unter Wärmeeinwirkung Aerosol bildenden Substanzen, durch die der heiße Luftstrom des Wärmetauschers durchgeführt wird, und mit einem Inhalatorauslass (4) zum Inhalieren des Aerosol/Luftgemischs, **dadurch gekennzeichnet, dass** die Füllkammer (8) ein inneres Kammergehäuse (13) aus einem gut wärmeleitenden Material aufweist und dass ein Wärmetauscher (3) verwendet wird, der sowohl in der Lage ist, den heißen Luftstrom zu erzeugen als auch das innere Kammergehäuse (13) direkt durch Wärmeleitung beheizt, so dass die Füllkammer (8) ihren Inhalt mittels Strahlungswärme beheizt.

2. Heißluftextraktionsinhalator nach Anspruch 1, **dadurch gekennzeichnet, dass** das innere Kammergehäuse (13) der Füllkammer (8) einstückig im Wärmetauscher fest integriert ist.

3. Heißluftextraktionsinhalator nach Anspruch 1, **dadurch gekennzeichnet, dass** die Füllkammer (8) mit dem inneren Kammergehäuse (13) vom Wärmetauscher (3) abnehmbar gestaltet und das innere Kammergehäuse (13) im aufgesetzten Zustand mit dem Wärmetauscher (3) thermisch leitend verbunden ist.

4. Heißluftextraktionsinhalator nach Anspruch 3, **dadurch gekennzeichnet, dass** das innere Kammergehäuse (13) der Füllkammer (8) stirnseitig an den Wärmetauscher (3) wahlweise anlegbar ist.

5. Heißluftextraktionsinhalator nach Anspruch 3 und 4, **dadurch gekennzeichnet, dass** die Füllkammer (8) zylindrisch ausgebildet und axial bewegbar in dem Inhalatorgehäuse (2) geführt ist.

6. Heißluftextraktionsinhalator nach Anspruch 3 bis 5, **dadurch gekennzeichnet, dass** die Füllkammer (8) umfangsseitig ein Gewinde (19) aufweist, das mit einem Gewinde (20) des Inhalatorgehäuses (2) zusammen wirkt.

7. Heißluftextraktionsinhalator nach Anspruch 3 bis 6, **dadurch gekennzeichnet, dass** die Füllkammer (8) einen thermisch isolierenden Betätigungshebel (16) zum Drehen gegenüber dem Inhalatorgehäuse (2) und dem Wärmetauscher (3) aufweist.

8. Heißluftextraktionsinhalator nach einem der vorhergehenden Ansprüche, **dadurch gekennzeichnet, dass** ein Inhalatorauslass (4) abnehmbar mit dem inneren Kammergehäuse (13) verbunden ist.

9. Heißluftextraktionsinhalator nach einem der vorhergehenden Ansprüche, **dadurch gekennzeichnet, dass** ein Inhalatorgehäuse (2) nach Art eines Handgerätes mit einem Griff (21) ausgebildet ist.

## Claims

1. A hot air extraction inhaler (1) including a heat exchanger (3), which is able to produce a hot air current, a filling chamber (8) for filling with substances which form an aerosol under the influence of heat, through which the hot air current from the heat exchanger is conducted, and including an inhaler outlet (4) for the inhalation of the aerosol/air mixture, **characterised in that** the filling chamber (8) includes an inner chamber housing (13) of a good thermally conductive material and that a heat exchanger (3) is used which not only is able to produce the hot air current but also heats the inner chamber housing (13) directly by thermal conduction so that the filling chamber (8) heats its contents by means of radiant heat.

2. A hot air extraction inhaler as claimed in Claim 1, **characterised in that** the inner chamber housing (13) of the filling chamber (8) is integrally permanently integrated into the heat exchanger.

3. A hot air extraction inhaler as claimed in Claim 1, **characterised in that** the filling chamber (8) with the inner chamber housing (13) is constructed to be removable from the heat exchanger (3) and the inner chamber housing (13) is thermally conductively connected to the heat exchanger (3) in the fitted condition.

4. A hot air extraction inhaler as claimed in Claim 3, **characterised in that** the inner chamber housing (13) of the filling chamber (8) is selectively mountable on the end surface of the heat exchanger (3).

5. A hot air extraction inhaler as claimed in Claims 3 and 4, **characterised in that** the filling chamber (8) is of cylindrical construction and is guided so as to be axially movable in the inhaler housing (2).

6. A hot air extraction inhaler as claimed in Claims 3 to 5, **characterised in that** the filling chamber (8) has on its periphery a screw thread (19), which cooperates with a screw thread (20) on the inhaler housing (2).

7. A hot air extraction inhaler as claimed in Claims 3 to 6, **characterised in that** the filling chamber (8) includes a thermally insulating actuating lever (16) for rotation with respect to the inhaler housing (2) and the heat exchanger (3).

8. A hot air extraction inhaler as claimed in one of the preceding claims, **characterised in that** an inhaler outlet (4) is removably connected to the inner chamber housing (13).

9. A hot air extraction inhaler as claimed in one of the preceding claims, **characterised in that** an inhaler housing (2) is constructed in the manner of a handheld unit with a handle (21).

## Revendications

1. Inhalateur à extraction par air chaud (1), comprenant un échangeur de chaleur (3) qui est en mesure de produire un courant d'air chaud, une chambre de remplissage (8) prévue pour être remplie de substances formant un aérosol sous l'effet de la chaleur, à travers laquelle le courant d'air chaud de l'échangeur de chaleur est conduit, et d'une sortie d'inhalateur (4) pour inhaler le mélange aérosol/air, **caractérisé en ce que** la chambre de remplissage (8) présente un boîtier de chambre intérieur (13) dans un matériau bon conducteur de la chaleur et qu'on utilise un échangeur de chaleur (3) qui à la fois est en mesure de produire le courant d'air chaud et chauffe directement le boîtier de chambre intérieur (13) par conduction thermique, de sorte que la chambre de remplissage (8) chauffe son contenu par chaleur rayonnante.

2. Inhalateur à extraction par air chaud selon la revendication 1, **caractérisé en ce que** le boîtier de chambre intérieur (13) de la chambre de remplissage (8) est fermement intégré d'une seule pièce dans l'échangeur de chaleur.

3. Inhalateur à extraction par air chaud selon la revendication 1, **caractérisé en ce que** la chambre de remplissage (8) avec le boîtier de chambre intérieur (13) est réalisée démontable de l'échangeur de chaleur (3) et le boîtier de chambre intérieur (13) est relié de manière thermiquement conductrice à l'échangeur de chaleur (3) dans l'état monté.

4. Inhalateur à extraction par air chaud selon la revendication 3, **caractérisé en ce que** le boîtier de chambre intérieur (13) de la chambre de remplissage (8) peut au choix être appliqué sur la face frontale de l'échangeur de chaleur (3).

5. Inhalateur à extraction par air chaud selon les revendications 3 et 4, **caractérisé en ce que** la chambre de remplissage (8) est de forme cylindrique et guidée mobile axialement dans le boîtier d'inhalateur (2).

6. Inhalateur à extraction par air chaud selon les revendications 3 à 5, **caractérisé en ce que** la chambre de remplissage (8) présente sur sa circonférence un filetage (19) qui coopère avec un filetage (20) du boîtier d'inhalateur (2).

7. Inhalateur à extraction par air chaud selon les revendications 3 à 6, **caractérisé en ce que** la chambre de remplissage (8) présente un levier de commande (16) thermiquement isolant pour la rotation par rapport au boîtier d'inhalateur (2) et à l'échangeur de chaleur (3).

8. Inhalateur à extraction par air chaud selon l'une des revendications précédentes, **caractérisé en ce qu'**une sortie d'inhalateur (4) est reliée de manière démontable au boîtier de chambre intérieur (13).

9. Inhalateur à extraction par air chaud selon l'une des revendications précédentes, **caractérisé en ce qu'**un boîtier d'inhalateur (2) est réalisé à la manière d'un appareil portatif doté d'une poignée (21).
